# EUROPEAN PATENT SPECIFICATION

(11) **EP 1 218 368 B1**
(45) Date of publication and mention of the grant of the patent: **23.05.2007**
(21) Application number: 00959287.4
(22) Date of filing: 18.08.2000
(51) Int. Cl.: C07D 321/00, A61K 31/335

(54) **COMPOSITIONS AND METHODS FOR MODULATING APOPTOSIS IN CELLS OVER-EXPRESSING bcl-2 FAMILY MEMBER PROTEINS**
ZUSAMMENSETUNGEN UND VERFAHREN ZUR MODULATION VON APOPTOSE IN CELLEN, DIE PROTEINE DER BC1-2-FAMILIE EXPRIMIEREN
COMPOSITIONS ET PROCEDES DE MODULATION DE L'APOPTOSE DANS DES CELLULES SUREXPRIMANT DES PROTEINES DE LA FAMILLE bcl-2

(30) Priority: 20.08.1999 US 149968 P
(43) Date of publication of application: 03.07.2002
(73) Proprietor: Fred Hutchinson Cancer Research Center, Seattle, WA 98109-1024 (US)
(72) Inventor: HOCKENBERY, David, M., Seattle, WA 98144 (US); SIMON, Julian, A., Seattle, WA 98118 (US); TZUNG, Shie-Pon, Issaquah, WA 98029 (US)
(74) Representative: Glawe, Delfs, Moll
(86) International application number: PCT/US2000/022891
(87) International publication number: WO 2001/014365

(56) References cited:
- WO-A-01/05769
- WO-A-99/40081
- US-A- 5 686 595
- US-A- 5 994 564
- PATENT ABSTRACTS OF JAPAN vol. 1995, no. 11, 26 December 1995 (1995-12-26) -& JP 07 196489 A (KOBE STEEL LTD), 1 August 1995 (1995-08-01)
- PATENT ABSTRACTS OF JAPAN vol. 017, no. 551 (C-1117), 5 October 1993 (1993-10-05) -& JP 05 155877 A (KAIYO BIO TECHNOL KENKYUSHO:KK), 22 June 1993 (1993-06-22)
- TOKUTAKE ET AL: "Inhibition of electron transport of rat-liver mitochondria by synthesized antimycin A analogs" BIOCHIMICA ET BIOPHYSICA ACTA. BIOENERGETICS, AMSTERDAM, NL, no. 1142, 1993, pages 262-268, XP002071909 ISSN: 0005-2728
- TZUNG S-P. ET AL.: "Antimycin A Mimics a BH3 Domain-Containing Peptide and Selectively Induces Apoptosis in Cell Lines Overexpressing Bcl-xl3" PROC.ACCR-NCI-EORTC INTERNATIONAL CONFERENCE, MOLECULAR TARGETS AND CANCER THERAPEUTICS, November 1999 (1999-11), pages 3815s-3816s, XP001145435

### SEQUENCE LISTING

<110> Fred Hutchinson Cancer Research Center
   Hockenbery, David M.
   Simon, Julian A.
   Tzung, Shie-Pon
<120> COMPOSITIONS AND METHODS FOR MODULATING APOPTOSIS IN CELLS OVER-EXPRESSING Bcl-2 FAMILY MEMBER PROTEINS
<130> 14538A-004610PC
<140>
   <141>
<150> 60/149,968
   <151> 1999-08-20
<160> 1
<170> PatentIn Ver. 2.1
<210> 1
   <211> 16
   <212> PRT
   <213> Artificial Sequence
<220>
   <223> Description of Artificial Sequence: Peptide
<400> 1
   Gly Gln Val Gly Arg Gln Leu Ala Ile Ile Gly Asp Asp Ile Asn Arg
    1               5                  10                  15

## Description

### BACKGROUND OF THE INVENTION

Mitochondria play a central role in mediating apoptosis in a number of apoptotic models (Kroemer et al., Immunol. Today 18:44-51 (1997); Zamzami et al., J. Exp. Med. 183:1533-44 (1996); Zamzami et al., J. Exp. Med. 182:367-77 (1995)). Cells induced to undergo apoptosis show an early disruption of mitochondrial transmembrane potential (ΔΨₘ) preceding other changes of apoptosis, such as nuclear fragmentation and exposure of phosphatidylserine on the outer plasma membrane. Isolated mitochondria or released mitochondrial products induce nuclear apoptosis in a cell-free reconstituted system (Liu et al., Cell 86:147-57 (1996); Newmeyer et al., Cell 79:353-64 (1994)).

Previous experiments indicated that the pre-apoptotic ΔΨm loss involves the opening of mitochondrial permeability transition (PT) pores, which are high-conductance channels at the inner mitochondrial membrane corresponding to mitochondrial megachannels identified by electrophysiological studies (Kroemer et al., supra; Zamzami et al. (1996), supra; Bernardi et al., Biochim. et Biophys. Acta 1275:5-9 (1996); Zoratti et al., Biochim. et Biophys. Acta 1241:139-76 (1995); Petit et al., FEBS Letters 396:7-13 (1996)). In fact, induction of PT is sufficient to provoke the full spectrum of apoptosis-associated changes. Conversely, agents that prevent opening of PT pores, such as bongkrekic acid, attenuate apoptosis (Kroemer et al., Immunol. Today 18:44-51 (1997); Zamzami et al., J. Exp. Med. 183:1533-44 (1996); Zamzami et al., FEBS Letters 384:53-57 (1996)).

Members of the evolutionarily conserved Bcl-2 family are important regulators of apoptotic cell death and survival. The proteins Bcl-2, Bcl-X_{L}, Bcl-w, A1 and Mcl-1 are death antagonists while Bax, Bak, Bad, Bcl-xs, Bid, and Bik are death agonists (Kroemer et al., Nature Med. 6:614-20 (1997)). Bcl-2 family member proteins are predominantly localized in the outer mitochondrial membrane, but are also found in the nuclear membrane and endoplasmic reticulum (Kroemer et al., supra).

Among Bcl-2 family member proteins, there are several conserved amino acid motifs, BH1-BH4. The pro-apoptotic members of the family, Bax and Bad, contain a BH3 domain that is sufficient to induce cell death (Chittenden et al., EMBO J. 14:5589-96 (1995); Hunter et al., J. Biol. Chem. 271:8521-24 (1996)). Interestingly, the BH3 domain is conserved in the anti-apoptotic proteins Bcl-2 and Bcl-x_{L}. Recently, it was reported that cleavage of Bcl-x_{L} and Bcl-2 in the loop domain removes the N-terminal BH4 domain and converts Bcl-x_{L} and Bcl-2 into a potent pro-death molecule (Cheng et al., Science 278:1966-68 (1997); Clem et al., Proc. Nat. Acad. Sci. USA 95:554-59 (1998)).

NMR structure analysis of a complex between Bcl-x_{L} and a 16 residue peptide encompassing the Bak BH3 domain demonstrated that the BH3 peptide, in an amphipathic alpha-helical configuration, binds with high affinity to the hydrophobic pocket created by the BH1, BH2 and BH3 domains of Bcl-x_{L} (Sattler et al., Science 275:983-86 (1997)). Leucine at position 1 of the BH3 domain core and aspartic acid at position 6 are believed to be critical residues for both heterodimerization and apoptosis induction. In further support of this conclusion, a number of "BH3 only" death promoters have been identified which have no similarity to Bcl-2 beyond their BH3 domain homology (Kelekar et al., Trends Cell Biol. 8:324-30 (1998)). These include Bik, Bim, Hrk, Bad, Blk, and Bid, which cannot homodimerize, but rely on binding to anti-apoptotic proteins such as Bcl-2 to induce cell death.

The exact mechanisms by which Bcl-2 prevents apoptosis remain elusive. In light of the importance of mitochondria in apoptosis and the mitochondrial location of Bcl-2, it appears that one major site where Bcl-2 interrupts apoptotic signals is at the level of mitochondria. It has been shown that Bcl-2 inhibits apoptosis by preventing mitochondrial permeability transition and by stabilizing ΔΨm (Zamzami et al., J. Exp. Med. 183:1533-44 (1996)). In the absence of Bcl-2, apoptogenic factors, such as cytochrome c and apoptosis inducing factor (AIF), are released from mitochondria in response to apoptotic triggers (Susin et al., J. Exp. Med. 184:1331-41 (1996); Kluck et al., Science 275:1132-36 (1997)). This release in turn leads to sequential caspase activation and results in nuclear and membrane changes associated with apoptosis.

Bc1-2 family members display a distinct tissue-specific expression. In adult human liver, Bc1-2 expression is confined to bile duct cells (Charlotte et al., Am. J. Pathol. 144:460-65 (1994)) and is absent in both normal and malignant hepatocytes. In contrast, expression of Bcl-x_{L} RNA and protein can be detected in adult quiescent hepatocytes and increases by 4 to 5 fold during the G1 phase of regenerating hepatocytes (Tzung et al., Am. J. Pathol. 150:1985-95 (1997)). Increased Bcl-x_{L} expression is also observed in hepatoma cell lines, such as HepG2.

Some diseases are believed to be related to the down-regulation of apoptosis in the affected cells. For example, neoplasias may result, at least in part, from an apoptosis-resistant state in which cell proliferation signals inappropriately exceed cell death signals. Furthermore, some DNA viruses, such as Epstein-Barr virus, African swine fever virus and adenovirus, parasitize the host cellular machinery to drive their own replication and at the same time modulate apoptosis to repress cell death and allow the target cell to reproduce the virus. Moreover, certain diseases, such as lymphoproliferative conditions, cancer (including drug resistant cancer), arthritis, inflammation, autoimmune diseases, and the like, may result from a down regulation of cell death signals. In such diseases, it would be desirable to promote apoptotic mechanisms.

Most cancer chemotherapeutic agents that are currently available target cellular DNA and induce apoptosis in tumor cells (Fisher et al., Cell 78:539-42 (1994)). A decreased sensitivity to apoptosis induction has emerged as an important mode of drug resistance. In particular, over-expression of Bcl-2 and Bcl-x_{L} confers resistance to multiple chemotherapeutic agents, including alkylating agents, antimetabolites, topoisomerase inhibitors, microtubule inhibitors and anti-tumor antibiotics, and may constitute a mechanism of clinical chemoresistance in certain tumors (Minn et al., Blood 86:1903-10 (1995); Decaudin et al., Cancer Res. 57:62-67 (1997)).

Neither Bcl-2 nor Bcl-x_{L}, however, protects cells from every apoptotic inducer. For example, over-expression of Bcl-2 offers little protection against Thy-1-induced thymocyte death and Fas-induced apoptosis (Hueber et al., J. Exp. Med. 179:785-96 (1994); Memon et al., J. Immunol. 15:4644-52 (1995)). At the mitochondrial level, Bcl-2 over-expressed in the outer mitochondrial membrane inhibits PT pore induction by t-butylhydroperoxide, protonophore and atractyloside, but not by calcium ions, diamide or caspase 1 (Zamzami et al., J. Exp. Med. 183:1533-44 (1996); Susin et al., J. Exp. Med. 186:25-37 (1997)). Thus, one class of mitochondrially-active agents may directly affect the mitochondrial apoptosis machinery while bypassing the site of Bcl-2 function and the protection offered by Bcl-2 family members. An agent of this type may potentially be useful in overcoming the multi-drug resistance imparted by Bcl-2 or Bel-X_{L} and are of great need in the art.

The antimycins constitute another class of mitochondrially-active agents. The antimycins generally comprise a N-formylamino salicylate moiety linked to a dilactone ring through an amide bond. The antimycins differ in the hydrophobic R groups attached to the dilactone ring opposite the amide bond. (See, e.g., Rieske, Pharm. Ther. 11:415-20 (1980).) For example, antimycin A₁ has a hexyl group at the 2 position of the dilactone ring while antimycin A₃ has a butyl group at that position.) Extensive literature has been published on the structure-activity relationship of the antimycins and their inhibition of cytochrome bc₁ (Miyoshi et al., Biochim. Biophys. Acta 1229:149-54 (1995); Tokutake et al., Biochim. Biophys. Acta 1142:262-68 (1993); Tokutake et al., Biochim. Biophys. Acta 1185:271-78 (1994)). The published structure of cytochrome bc₁ complex with bound antimycin A₁ reveals that antimycin A₁ occupies a position in the Qi ubiquinone binding site on cytochrome b (Xia et al., Proc. Nat. Acad. Sci. USA 94:11399-404 (1997)). The antimycins generally inhibit mitochondrial respiration, which suggests that the differences in the hydrophobic R groups on the dilactone ring are not critical for cytochrome b binding. Mutagenesis and structure-activity studies of antimycin A demonstrate that the cytochrome bc₁-inhibitory activity is highly dependent on the N-formylamino salicylic acid moiety (Tokutake et al. (1994), supra). Methylation of the phenolic hydroxyl or modification of the N-formylamino group both significantly reduce the ability of antimycin A to bind to and inhibit cytochrome bc₁. Methylation of the phenolic hydroxyl diminishes inhibitory activity by 2.5 logs. Substitution of the formylamino group with acetylamino and propylamino groups at the 3-position reduce cytochrome bc₁ activity by 1.2 and 2.4 logs, respectively. Thus, the N-formylamino salicylate moiety is generally understood to be important for binding of the antimycins to cytochrome b.

Two antimycins, antimycin A₁ and A₃, have recently been discovered to inhibit the activity of the anti-apoptotic Bcl-2 family member proteins, Bcl-2 or Bel-X_{L}. Thus, these molecules potentially useful compounds for the medical profession and patients suffering from proliferative disease and other diseases where apoptosis is inappropriately regulated. The antimycins are toxic, however, because they also inhibit mitochondrial respiration. There is a critical need, therefore, for derivatives of the antimycins that are effective in inducing apoptosis in cells where apoptosis is inappropriately regulated while exhibiting reduced inhibition of mitochondrial respiration.

### SUMMARY OF THE INVENTION

The present invention is defined in the claims and is based on the surprising discovery that the antimycins can inhibit the activity of anti-apoptotic Bcl-2 family member proteins, such as Bcl-2 or Bcl-x_{L}. The invention is further based on the discovery that mitochondrial respiratory inhibitory activity, of the antimycins, is separable from the inhibition of the Bcl-2 family member proteins.

The present invention provides the use of agents comprising derivatives of antimycins that modulate apoptosis by binding to a Bcl-2 family member protein for the manufacture of a medicament as defined in claim 1. These agents exhibit reduced binding to cytochrome B (or the cytochrome bc₁ complex, hereafter referred to as "cytochrome B") as compared with non-derivatized antimycins. In one embodiment, the agent preferentially induces apoptosis in cells that over-express an anti-apoptotic Bcl-2 family member protein. In another embodiment, the agent is substantially non-toxic to cells that do not over-express the anti-apoptotic Bcl-2 family member protein. The agent typically inhibits the activity of an anti-apoptotic Bcl-2 family member protein by binding to the hydrophobic pocket formed by the BH1, BH2 and BH3 domains of the protein.

The agents comprises derivatives of an antimycin, or a portion thereof, such as chemical modification of the dilactone moiety (i.e., the 4,9-dioxo-1,5-dioxanan-7-yl ester moiety). In a preferred embodiment, the antimycin derivative comprises at least two chemical modifications. The modification decreases the affinity of the antimycin derivative for cytochrome B and increases the affinity of the antimycin derivative for a Bcl-2 family member protein.

In another aspect, the invention provides the use of pharmaceutical compositions comprising the agent for treating a subject in which a cell over-expresses an anti-apoptotic Bcl-2 family member protein. Such compositions are useful for treating apoptosis-associated diseases or conditions as defined in claim 1.

### BRIEF DESCRIPTION OF THE DRAWINGS

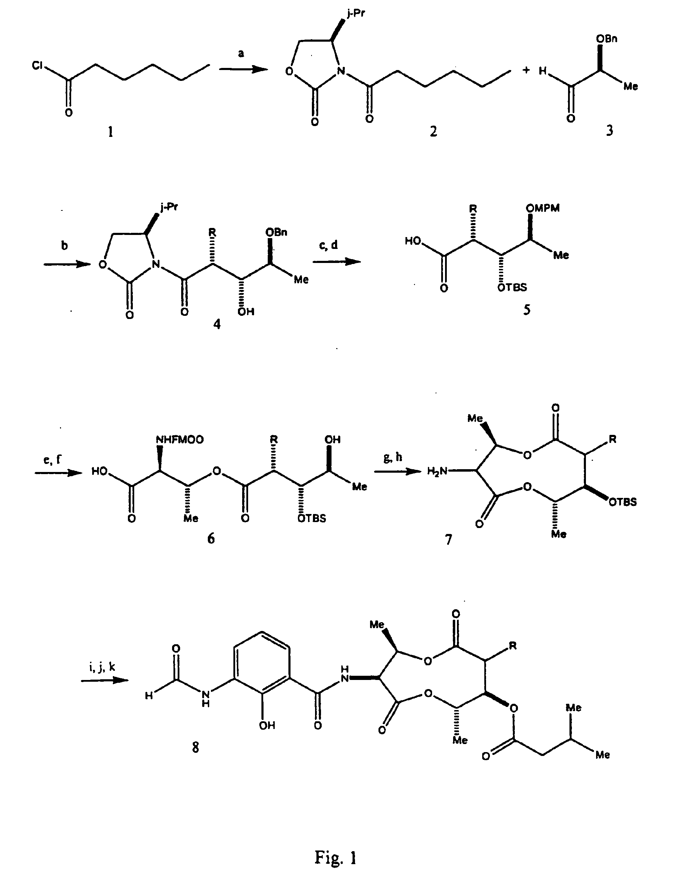

Figure 1 depicts a general scheme for the chemical synthesis of antimycin A₃, and for the synthesis of derivatives of antimycins.

### DESCRIPTION OF THE SPECIFIC EMBODIMENTS

Prior to setting forth the invention in more detail, it may be helpful to a further understanding thereof to set forth definitions of certain terms as used hereinafter.

### Definitions:

The term "apoptosis" refers to a regulated network of biochemical events which lead to a selective form of cell suicide, and is characterized by readily observable morphological and biochemical phenomena, such as the fragmentation of the deoxyribonucleic acid (DNA), condensation of the chromatin, which may or may not be associated with endonuclease activity, chromosome migration, margination in cell nuclei, the formation of apoptotic bodies, mitochondrial swelling, widening of the mitochondrial cristae, opening of the mitochondrial permeability transition pores and/or dissipation of the mitochondrial proton gradient.

The term "antimycins" refers to the antimycins A₀ (a-d), A₁, A₂, A₃, A₄, A₅, A₆, kitamycin A and B, urauchimycin B, deisovaleryl blastomycin, and dehexyl-deisovaleryloxy antimycin A. The antimycins are generally represented by the following formula I, and have the absolute configuration [2R, 3R, 4S, 7S, 8R]:

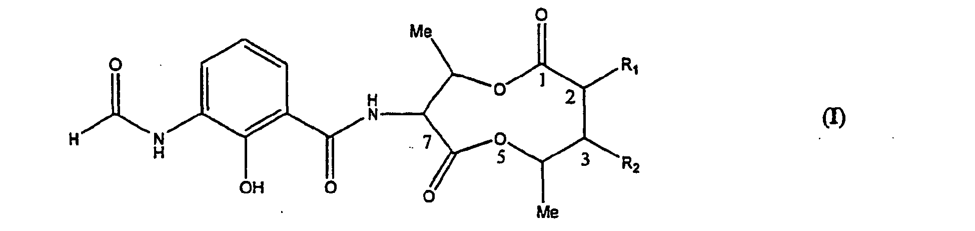

The groups at positions R1 and R2 vary as follows:

**Table 1**

| Name | R₁ | R₂ |
|---|---|---|
| antimycin A₀₍ₐ₎ | hexyl | hexanoic acid |
| antimycin A_{0(b)} | butyl | heptanoic acid |
| antimycin A_{0(c)} | octyl | butanoic acid |
| antimycin A_{0(d)} | heptyl | isovaleric acid |
| antimycin A₁ | hexyl | isovaleric acid |
| antimycin A₂ | hexyl | butanoic acid |
| antimycin A₃ | butyl | isovaleric acid |
| antimycin A₄ | butyl | butanoic acid |
| antimycin A₅ | ethyl | isovaleric acid |
| antimycin A₆ | ethyl | butanoic acid |
| kitamycin A | hexyl | hydroxyl |
| kitamycin B | isohexyl | hydroxyl |
| urauchimycin B | isohexyl | hydroxyl |
| deisovalerylblastomycin | butyl | hydroxyl |
| dehexyl-deisovalerylblastomycin | hydrogen | hydrogen |

The term "antimycin derivative" refers to a chemical modification of an antimycin, by which one or more atoms of an antimycin are removed or substituted, or new atoms are added. An "antimycin derivative" further includes portions of an antimycin as well as chemical modifications thereof, and chiral variants of an antimycin.

The term "agent" is used herein to denote a chemical compound, or a mixture of chemical compounds, salts and solvates thereof, and the like, which are capable of modulating the biological activity of a Bcl-2 family member protein. An agent typically comprises an antimycin derivative.

The term "preferentially induce" apoptosis refers to at least a 5-fold greater stimulation of apoptosis, at a given concentration of an agent, in cells that over-express a Bcl-2 family member protein as compared with cells that do not over-express the Bcl-2 family member protein (e.g., a 5-fold greater LD₅₀ or IC₅₀).

The term "substantially non-toxic" refers to an agent that induces apoptosis in at least about 50 percent of cells that over-express a Bcl-2 family member protein, but does not induce apoptosis in more than about 5%, more preferably less than 1%, of cells that do not over-express the Bcl-2 family member protein.

The term "Bcl-2 family member protein(s)" refers to an evolutionarily conserved family of proteins characterized by having one or more amino acid homology domains, BH1, BH2, BH3, and/or BH4. The Bcl-2 family member proteins include Bcl-2, Bcl-x_{L}, Bcl-w, A1, Mcl-1, Bax, Bak, Bad, Bcl-xs and Bid. The "Bcl-2 family member proteins" further include those proteins, or their biologically active fragments, that are at least 70% similar in amino acid sequence to a Bcl-2 family member protein.

The term "anti-apoptotic Bcl-2 family member protein" refers to Bcl-2, Bcl-x_{L}, Bcl-w, A1, Mcl-1, and other proteins characterized by having one or more amino acid homology domains, BH1, BH2, BH3, and/or BH4, and that promote cell survival by attenuating or inhibiting apoptosis. The "anti-apoptotic Bcl-2 family member proteins" further include those proteins, or their biologically active fragments, that are at least 70% similar in amino acid sequence to an anti-apoptotic Bcl-2 family member protein.

The terms "biologically active" or "biological activity" refer to the ability of a molecule to modulate apoptosis, such as by binding to a Bcl-2 family member protein. A biologically active molecule can modulate apoptosis by causing a change in the mitochondrial proton gradient, by causing a change in mitochondrial swelling or the morphological characteristics of mitochondria, by affecting the release of a reporter molecule, such as, for example, rhodamine 123 or calcein, from mitochondria or vesicles comprising a pore-forming anti-apoptotic Bcl-2 family member protein or by causing any other morphological change associated with apoptosis.

Antimycin derivatives can be prepared by chemically modifying an antimycin according to standard chemical methods. For example, the hydroxyl group on the salicylate moiety of antimycin A₃ can be modified using a primary alkyl halide or diazomethane to form a 2-alkoxy ether antimycin derivative (e.g., 2-methoxy ether antimycin A₃). An antimycin can also be modified by acetylation.

Alternatively, antimycin derivatives can be prepared by de novo ("total") chemical synthesis. For example, Shimano et al. (Tetrahedron 54:12745-74 (1998), have devised a total synthetic method for the related antifungal dilactones UK-2A and UK-3A. This total synthesis can be used to prepare antimycin derivatives. According to this method, antimycin A₃ can be modeled as comprising three structural units: N-formyl-3-aminosalicylic acid, L-threonine, and the dilactone moiety. (See formulae III-V, respectively.) 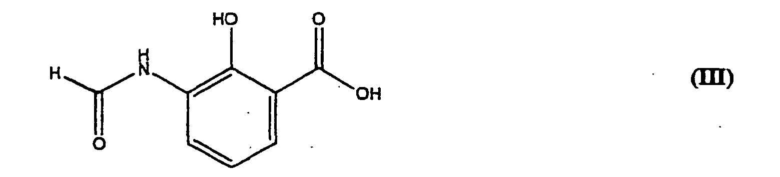 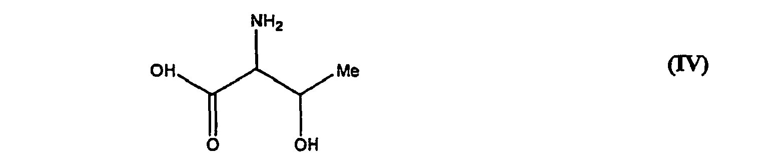 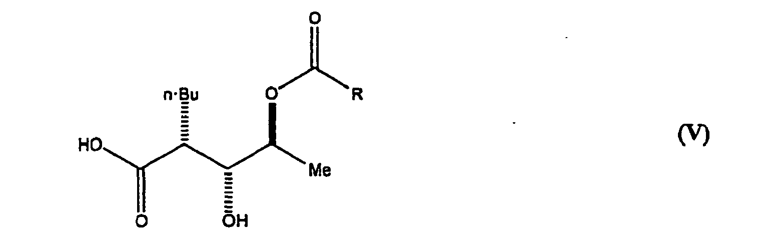 Antimycin A₃ can be synthesized by joining these structural units. Derivatives of one or more of these structural units can be chemically linked to form antimycin derivatives.

Libraries of antimycin derivatives can also be prepared by rational design. (See generally, Cho et al., Pac. Symp. Biocompat. 305-16 (1998); Sun et al., J. Comput. Aided Mol. Des. 12:597-604 (1998)). For example, libraries of antimycin derivatives can be prepared by syntheses of combinatorial chemical libraries (see generally DeWitt et al., Proc. Nat. Acad. Sci. USA 90:6909-13 (1993); International Patent Publication WO 94/08051; Baum, Chem. & Eng. News, 72:20-25 (1994); Burbaum et al., Proc. Nat. Acad. Sci. USA 92:6027-31 (1995); Baldwin et al., J. Am. Chem. Soc. 117:5588-89 (1995); Nestler et al., J. Org. Chem. 59:4723-24 (1994); Borehardt et al., J. Am. Chem. Soc. 116:373-74 (1994); Ohlmeyer et al., Proc. Nat. Acad. Sci. USA 90:10922-26 (1993); and Longman, Windhover's In Vivo The Business & Medicine Report 12:23-31 (1994)).

The following articles describe methods for selecting starting molecules and/or criteria used in their selection: Martin et al., J. Med. Chem. 38:1431-36 (1995); Domine et al., J. Med. Chem., 37:973-80 (1994); Abraham et al., J. Pharm. Sci. 83:1085-100 (1994).

A "combinatorial library" is a collection of compounds in which the compounds comprising the collection are composed of one or more types of subunits. The subunits can be selected from natural or unnatural moieties, including dienes, benzene compounds, cycloalkanes, lactones, dilactones, amino acids, alkanes, and the like. The compounds of the combinatorial library differ in one or more ways with respect to the number, order, type or types of modifications made to one or more of the subunits comprising the compounds. Alternatively, a combinatorial library may refer to a collection of "core molecules" which vary as to the number, type or position of R groups they contain and/or the identity of molecules composing the core molecule. The collection of compounds is generated in a systematic way. Any method of systematically generating a collection of compounds differing from each other in one or more of the ways set forth above is a combinatorial library.

A combinatorial library can be synthesized on a solid support from one or more solid phase-bound resin starting materials. The library can contain five (5) or more, preferably ten (10) or more, organic molecules which are different from each other (i.e., five (5) different molecules and not five (5) copies of the same molecule). Each of the different molecules (different basic structure and/or different substituents) will be present in an amount such that its presence can be determined by some means (e.g., can be isolated, analyzed, detected with a binding partner or suitable probe). The actual amounts of each different molecule needed so that its presence can be determined can vary due to the actual procedures used and can change as the technologies for isolation, detection and analysis advance. When the molecules are present in substantially equal molar amounts, an amount of 100 picomoles or more can be detected. Preferred libraries comprise substantially equal molar amounts of each desired reaction product and do not include relatively large or small amounts of any given molecules so that the presence of such molecules dominates or is completely suppressed in any assay.

Combinatorial libraries are generally prepared by derivatizing a starting compound onto a solid-phase support (such as a bead). In general, the solid support has a commercially available resin attached, such as a Rink or Merrifield Resin. After attachment of the starting compound, substituents are attached to the starting compound. For example, a benzene compound can be bound to a support via a Rink resin. The benzene ring is reacted simultaneously with an amide, such as a N-formylamino, N-acetylamino, N-propionylamino, and the like. Alternatively, the starting compound can comprise the dilactone moiety, or a precursor thereof. Substituents are added to the starting compound, and can be varied by providing a mixture of reactants comprising the substituents. Examples of suitable substituents include, but are not limited to, the following:
(1) hydrocarbon substituents, that is, aliphatic (e.g., alkyl or alkenyl), alicyclic (e.g., cycloalkyl, cycloalkenyl) substituents, aromatic, aliphatic and alicyclic-substituted aromatic nuclei, and the like, as well as cyclic substituents;
(2) substituted hydrocarbon substituents, that is, those substituents containing nonhydrocarbon radicals which do not alter the predominantly hydrocarbon substituent; those skilled in the art will be aware of such radicals (e.g., halo (especially chloro and fluoro), alkoxy, mercapto, alkylmercapto, nitro, nitroso, sulfoxy, and the like);
(3) hetero substituents, that is, substituents which will, while having predominantly hydrocarbyl character, contain other than carbon atoms. Suitable heteroatoms will be apparent to those of ordinary skill in the art and include, for example, sulfur, oxygen, nitrogen, and such substituents as pyridyl, furanyl, thiophenyl, imidazolyl, and the like. Heteroatoms, and typically no more than one, will be present for each carbon atom in the hydrocarbon-based substituents. Alternatively, there may be no such radicals or heteroatoms in the hydrocarbon-based substituent and it will, therefore, by purely hydrocarbon.

In one embodiment, a combinatorial library of derivatives of antimycins is prepared. For example, the starting compound can be a precursor of the dilactone moiety. A combinatorial library of the dilactone is synthesized using the Shimano synthesis (infra) while varying the substituents added at each step of the synthesis. Optionally, following lactonization, the threonine and salicylic acid moieties, or derivatives thereof, are added to the library.

Methods of making combinatorial libraries are known in the art, and include the following: U.S. Patent Nos. 5,958,792; 5,807,683; 6,004,617; 6,077,954.

Agents of the present invention are useful for treating cells in which the cell death signal is down regulated and the affected cell has an inappropriately diminished propensity for cell death, which is referred to herein as being in a "decreased apoptotic state." The invention further provides medicaments for the administration to a subject of a therapeutically effective amount of an agent to treat an apoptosis-associated disease in which it is desirable to induce apoptosis in certain types of cells, such as virus-infected or autoantibody-expressing cells. Typically, the agent is substantially purified prior to administration. The subject can be an animal, including but not limited to, cows, pigs, horses, chickens, cats, dogs, and the like, and is typically a mammal, and in a particular embodiment human. In another specific embodiment, a non-human mammal is the subject.

Various delivery systems are known and can be used to administer an agent, such as, for example, encapsulation in liposomes, microparticles, microcapsules, recombinant cells capable of expressing the agent, receptor-mediated endocytosis (see, e.g., Wu and Wu, J. Biol. Chem. 262:4429-32 (1987)), and the like. The agents administered as therapeutic or pharmaceutical compositions by any suitable route known to the skilled artisan including, for example, intravenous, subcutaneous, intramuscular, intradermal, transdermal, intrathecal, intracerebral, intraperitoneal, epidural and oral routes. Administration can be either rapid as by injection or over a period of time as by slow infusion or administration of slow release formulations. For treating tissues in the central nervous system, administration can be by injection or infusion into the cerebrospinal fluid (CSF). When it is intended that an agent be administered to cells in the central nervous system, administration can be with one or more other components capable of promoting penetration of the agent across the blood-brain barrier. In addition, it can be desirable to introduce an agent into the target-tissue by any suitable route, including intravenous and intrathecal injection. Pulmonary administration can also be employed, such as, for example, by use of an inhaler or nebulizer, and formulation of the agent with an aerosolizing agent.

Pharmaceutical compositions can also be administered orally in any orally acceptable dosage form including, but not limited to, capsules, tablets, caplets, lozenges, aqueous suspensions or solutions. In the case of tablets for oral use, carriers which are commonly used include lactose and corn starch. Lubricating aids, such as magnesium stearate, are also typically added. For oral administration in a capsule form, useful diluents include lactose and dried corn starch. When aqueous suspensions are required, the agent can be combined with emulsifying and suspending aids. If desired, certain sweeteners, flavorants or colorants can also be used.

In a specific embodiment, it can be desirable to administer the agent locally to the area in need of treatment; this administration can be achieved by, for example, and not by way of limitation, local infusion during surgery, topical application (e.g., in conjunction with a wound dressing after surgery), by injection, by means of a catheter, by means of a suppository, or by means of an implant, the implant being of a porous, non-porous, or gelatinous material, including membranes such as silastic membranes, or fibers. In one embodiment, administration can be by direct injection at the site (or former site) of a malignant tumor or neoplastic or pre-neoplastic tissue.

In another embodiment, the agent can be delivered in a vesicle, in particular a liposome (see, e.g., Langer, Science 249:1527-33 (1990); Treat et al., In Liposomes in the Therapy of Infectious Disease and Cancer, Lopez-Berestein and Fidler (eds.), Liss, New York, pp. 353-65 (1989); Lopez-Berestein, supra, pp. 317-27).

In yet another embodiment, the agent can be delivered in a controlled release system. In one embodiment, a pump can be used (see, e.g., Langer, supra; Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used (see, e.g., Medical Applications of Controlled Release, Langer and Wise (eds.), CRC Pres., Boca Raton, Florida (1974); Controlled Drug Bioavailability. Drug Product Design and Performance, Smolen and Ball (eds.), Wiley, New York (1984); Ranger and Peppas, J. Macromol. Sci. Rev. Macromol. Chem. 23:61 (1983); see also Levy et al., Science 228:190 (1985); During et al., Ann. Neurol. 25:351 (1989); Howard et al., J. Neurosurg. 71:105 (1989)). In yet another embodiment, a controlled release system can be placed in proximity of the therapeutic target, thus requiring only a fraction of the systemic dose (see, e.g., Goodson, Medical Applications of Controlled Release, supra, Vol. 2, pp. 115-138 (1984)). Other controlled release systems are discussed in, for example, the review by Langer (Science 249:1527-33 (1990)).

The present invention also provides the use of pharmaceutical compositions. Such compositions comprise a therapeutically effective amount of an agent, and a pharmaceutically acceptable carrier. The term "pharmaceutically acceptable" means approved by a regulatory agency of the Federal or a state government or listed in the U.S. Pharmacopeia or other generally recognized pharmacopeia for use in animals, and more typically in humans. The term "carrier" refers to a diluent, adjuvant, excipient, stabilizer, or vehicle with which the agent is formulated for administration. Pharmaceutical carriers can be sterile liquids, such as water and oils, including those of petroleum, animal, vegetable or synthetic origin, such as peanut oil, soybean oil, mineral oil, sesame oil, and the like. Water is a typical carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like. The composition, if desired, can also contain minor amounts of wetting or emulsifying agents, or pH buffering agents. Pharmaceutical compositions can take the form of solutions, suspensions, emulsion, tablets, pills, capsules, powders, sustained-release formulations, and the like. The composition can be formulated as a suppository, with traditional binders and carriers such as triglycerides.

Oral formulations can include standard carriers such as pharmaceutical grades of mannitol, lactose, starch, magnesium stearate, sodium saccharine, cellulose, magnesium carbonate, and the like. Examples of suitable pharmaceutical carriers are described in, for example, Remington's Pharmaceutical Sciences, by E.W. Martin. Such compositions will contain a therapeutically effective amount of the agent, typically in purified form, together with a suitable amount of carrier so as to provide a formulation proper for administration to the subject. The formulation should suit the mode of administration.

In one embodiment, the agent is formulated in accordance with routine procedures as a pharmaceutical composition adapted for intravenous administration to human beings. Typically, compositions for intravenous administration are solutions in sterile isotonic aqueous buffer. Where necessary, the composition can also include a solubilizing agent and a local anesthetic to ease pain at the site of the injection. Generally, the ingredients are supplied either separately or mixed together in unit dosage form. For example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

The agents can be formulated as neutral or salt forms. Pharmaceutically acceptable salts include those formed with free amino groups such as those derived from hydrochloric, phosphoric, acetic, oxalic, tartaric acids, and the like, and those formed with free carboxyl groups such as those derived from sodium, potassium, ammonium, calcium, ferric hydroxides, isopropylamine, triethylamine, 2-ethylamino ethanol, histidine, procaine, and the like.

The amount of the agent that is combined with the carrier to produce a single dosage form will vary, depending upon the nature of that agent and the composition of the dosage form. It should be understood, however, that a specific dosage and treatment regime for any particular patient, or disease state will depend upon a variety of factors, including the age, body weight, general health, sex, diet, time of administration, rate of excretion, drug combination, the judgment of the treating physician, and the severity of the particular disease being treated. The amount of active agent will also depend upon the specific activity of the agent and whether the agent is co-administered with any other therapeutic or prophylactic ingredients. Dosage levels of between about 0.001 and about 100 mg/kg body weight per day, typically between about 0.1 and about 10 mg/kg body weight per day of the active agent are useful.

The medicament may be in the form of a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The following examples are provided merely as illustrative of various aspects of the invention and shall not be construed to limit the invention in any way.

### EXAMPLE 1

Studies of cellular respiration, ATP levels and reactive oxygen species in antimycin A-treated cell lines strongly suggested that the observed differences in cell viability could not be explained by the known effects of antimycin A on mitochondrial electron transfer or oxidative phosphorylation. To definitively address which activities of antimycin A are involved in the selective cell death of cell over-expressing Bel-X_{L}, the structure-activity relationship for antimycin A₃ as an inhibitor of Bcl-x_{L} pore activity was determined.

In this example, two derivatives of antimycin A₃ were prepared, antimycin A₃ methyl ether (2-methoxy ether antimycin A₃) and phenacyl ether antimycin A₃ (comparative example). The structure of antimycin A₃ was shown above (formula (I), where R₁ is a butyl group). (See also van Tamelen et al, J. Am. Chem. Soc. 83:1639 (1961)). Antimycin A₃ methyl ether has the following formula (VI) and an absolute configuration of [2R, 3R, 4S, 7S, 8R]:

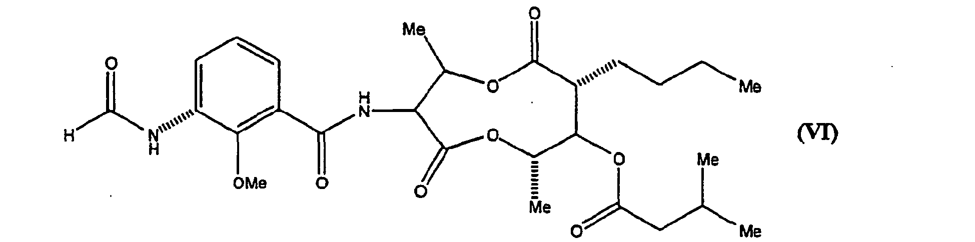

Antimycin A₃ methyl ether is prepared directly from antimycin A₃ as follows: Briefly, antimycin A₃ (14.0 mg) was dissolved in ethyl ether and a stream of diazomethane was passed through the reaction mixture until the yellow color persisted. The reaction mixture was treated with acetic acid until it became colorless. The mixture was reduced to dryness under reduced pressure and chromatographed on silica gel to yield 14.3 mg of antimycin A₃ methyl ether. The resulting product was characterized by NMR, infrared spectroscopy and mass spectroscopy.

The phenacyl ether derivative of antimycin A₃ was prepared as follows: A solution of antimycin A₃ (5.7 mg, 10.95 mmol) in dry acetonitrile was treated with phenacyl bromide (4.4 mg, 21.9 mmol) and powdered potassium carbonate (6.0 mg, 43.8 mmol). The mixture was allowed to stir at room temperature for 18 hours. The reaction mixture was applied directly to a silica gel chromatography column. The product was eluted with 20% ethyl acetate/hexane to yield 5.4 mg (78%) of the product as a colorless oil. The resulting product was characterized by NMR, infrared spectroscopy and mass spectroscopy.

### EXAMPLE 2

The antimycin A₃ methyl ether derivative prepared in Example 1 was studied to determine its affect on the apoptotic pathway in cells over-expressing Bcl-x_{L}. The methyl ether derivative was previously shown to be inactive as an inhibitor of cytochrome bc₁. (See, e.g., Miyoshi et al., Biochim Biophys Acta 1229:149-54 (1995); Takotake et al., Biochim Biophys Acta 1185:271-78 (1994).) The methyl ether also has a negligible effect on cellular O₂ consumption compared to the original antimycin A₃ compound. TABX2S (over-expressing Bcl-x_{L}), TAMH.neo (control) and TABX1A (antisense) cell lines were treated with 2-methoxy antimycin A₃. These cell lines exhibited a pattern of preferential cytotoxicity for cells over-expressing Bcl-x_{L}, but not for control cells. This pattern was similar to that to antimycin A₃ treatment of these cell lines, indicating that the effect of this antimycin derivative on cellular respiration was separable from that on apoptosis.

To confirm this data, assays were also performed with mitochondrial fractions from each cell line using the mitochondrial probe JC-1. Mitochondria from cells over-expressing Bel-X_{L} (TABX2S cells) were strongly depolarized after addition of the 2-methoxy derivative at a concentration of 2 µg/ml. As observed for the parent compound, antimycin A₃, mitochondria with normal levels of Bcl-x_{L} expression were not affected by the 2-methoxy analog.

Finally, the 2-methoxy antimycin A₃ derivative was shown to bind recombinant Bcl-2. The 2-methoxy antimycin A₃ derivative is non-fluorescent due to the additional electrophilic substituent on the benzene ring. Thus, binding of 2-methoxy antimycin A₃ to the Bcl-2 protein can be measured in a competitive binding assay by monitoring fluorescence from antimycin A₃. For these experiments, antimycin A₃ (2 µM) and either 2-methoxy ether antimycin A₃ or phenacyl ether antimycin A₃ (2 µM) were added simultaneously to Bcl-2 polypeptide (3 µM) and allowed to equilibrate for 7.5 minutes at 22.5° C before measuring the fluorescence intensity of antimycin A₃. The fluorescence of a prebound antimycin A₃- recombinant Bcl-2 complex decreased exponentially with the addition of 2-methoxy antimycin A₃, indicating competition for the antimycin A₃ binding site on Bcl-2. As an additional control for binding specificity, the effect of the phenacyl ether derivative of antimycin A₃ was also tested. Although of similar hydrophobicity, the phenacyl ether derivative did not displace antimycin A₃ from Bcl-2. These results strongly suggest that the cellular and mitochondrial sensitivity to antimycin A₃ in Bcl-x_{L} expressing cell lines results from direct binding of antimycin A₃ to Bcl-X_{L} protein. Furthermore, the 2-methoxy ether antimycin A₃ derivative inhibited Bcl-x_{L} pore formation in a liposome permeability assay almost as well as antimycin A₃.

The results demonstrate that the antimycins have two structurally distinguishable protein-binding activities, one for binding to cytochrome bc₁, and the other for binding to Bcl-2 family member proteins, and that these activities are separable.

### EXAMPLE 3

The total synthesis of antimycin A₃ is carried out essentially as described by Shimano for the related dilactones UK-2A and UK-3A (Shimano, Tetrahedron 54:12745-74 1998). Briefly, antimycin A₃ is composed of three structural units: an N-formyl-3-aminosalicylic acid, L-threonine and 2-butyl-3,4-dihydroxypentanoic acid. Of the three structural components, N-formyl-3-aminosalicylic acid and L-threonine are commercially available. The dihydroxy pentanoic acid is prepared in a four-step reaction sequence starting with caproyl chloride. Referring to Figure 1, caproyl chloride is reacted with the Evans valine-derived oxazolidinone, (R)-4-isopropyloxazolidin-2-one, and n-butylLi (Step a). The resulting adduct (2) is reacted by aldol condensation with a chiral aldehyde derived from (S)-(-)-lactic acid (3) in the presence of dibutyl-BOTf and triethylamine (Step b). The 4-hydroxyl group of the resulting adduct (4) is protected as a t-butyldimethylsilyl ether (using TBS chloride and DIEA), followed by peroxide-mediated hydrolysis (using hydrogen peroxide and lithium hydroxide) of the chiral auxiliary to yield the differentially protected dihydroxy pentanoic acid (5) (Steps c and d). Differential protection of the two secondary alcohols allows for the incorporation of various carboxylic acids at the 3 position of the lactone. The carboxylic acid is coupled to N-FMOC-L-threonine benzyl ester with BOP-chloride and DMAP (Step e). Removal of the two benzyl protecting groups with H₂ and Pd/O will yield the dilactone seco-acid (6) (Step f). Lactonization occurs using a BOP-Cl mediated ester-forming reaction with DMAP (Step g). Diethylamine is used to remove the FMOC protecting group to yield the dilactone (7) (Step h). N-formyl-3-amine salicylic acid is coupled to the dilactone using standard carbodiimide chemistry (Step i). In particular, the dilactone- is combined with N-formyl-3-aminosalicylic acid using EDCl and HOBT, followed by treatment with TBAF. The final elaboration of the derivatized antimycin A3 structure is accomplished by fluoride-mediated removal of the silyl protecting group and coupling of the desired acid chloride (e.g., isovaleryl chloride and DIEA) (Steps j and k).

### EXAMPLE 4

To prepare derivatives of antimycin A₃ that are modified in the isovalerate moiety (i.e., R₂) of the dilactone, the total synthesis of antimycin A₃ (as described in Example 3) is conducted with the following modifications: After dilactonization, the isovaleryl chloride is substituted by another acyl chloride, such as acetyl chloride, butyryl chloride, and the like.

### EXAMPLE 5

To prepare derivatives of antimycin A₃ in which the butyl group (i.e., R₁) on the dilactone is substituted with another R group, the total synthesis of antimycin A₃ (as described in Example 3) is conducted with the following modifications: The caproyl chloride of step 1 is substituted with another acyl chloride, such as propionyl chloride or another linear or branched acyl chloride.

## Claims

1. Use of a therapeutically effective amount of an antimycin derivative for the manufacture of a medicament for the treatment of an apoptosis-associated disease selected from the group consisting of neoplasias, lymphoproliferative conditions, cancer and drug resistant cancer, wherein said antimycin derivative is selected from the group consisting of antimycin A₀₍ₐ₎, antimycin A_{0(b)}, antimycin A_{0(c)}, antimycin A_{0(d)}, antimycin A₁, antimycin A₂, antimycin A₃, antimycin A₄, antimycin A₅, antimycin A₆, kitamycin A, kitamycin B, urauchimycin B, deisovalerylblastomycin, dehexyl-deisovalerylblastomycin; with the proviso that the hydroxyl group at the benzene ring is replaced with a methoxy group.

2. The use of claim 1, wherein the antimycin derivative is 2-methoxy ether antimycin A or A₃.

3. The use of claim 1 or 2, wherein the medicament further comprises a pharmaceutical carrier.

4. The use of claim 1, 2, or 3, wherein the medicament is formulated for intravenous, subcutaneous, intramuscular, intradermal, transdermal, intrathecal, intracerebral, intraperitoneal, epidural or oral administration.

## Patentansprüche

1. Verwendung einer therapeutisch wirksamen Menge eines Antimycinderivates zur Herstellung eines Medikaments zur Behandlung von Krankheiten, die mit Apoptose in Zusammenhang stehen, die ausgewählt sind aus der Gruppe bestehend aus Neoplasien, lymphoproliferativen Zuständen, Krebs und Medikamenten-resistenter Krebs,
wobei das Antimycinderivat ausgewählt ist aus der Gruppe bestehend aus Antimycin A₀₍ₐ₎, Antimycin A_{0(b)}, Antimycin A_{0(c)}, Antimycin A_{0(d)}, Antimycin A₁, Antimycin A₂, Antimycin A₃, Antimycin A₄, Antimycin A₅, Antimycin A₆, Kitamycin A, Kitamycin B, Urauchimycin B, Deisovalerylblastomycin, Dehexyl-Deisovalerylblastomycin;
mit der Maßgabe, dass die Hydroxylgruppe an dem Benzolring ersetzt ist durch eine Methoxygruppe.

2. Verwendung nach Anspruch 1, wobei das Antimycinderivat 2-Methoxy Ether Antimycin A oder A₃ ist.

3. Verwendung nach Anspruch 1 oder 2, wobei das Medikament zusätzlich einen pharmazeutischen Träger umfasst.

4. Verwendung nach einem der Ansprüche 1, 2 oder 3, wobei das Medikament formuliert ist für intravenöse, subcutane, intramuskuläre, intradermale, transdermale, intrathecale, intracerebrale, intraperitoneale, epidurale oder orale Verabreichung.

## Revendications

1. Utilisation d'une quantité thérapeutiquement efficace d'un dérivé antimycine pour la fabrication d'un médicament pour le traitement de maladies associées à l'apoptose choisi parmi le groupe consistant en : les conditions néoplasiques, lymphoprolifératives, le cancer, et le cancer pharmacorésistant, choisi parmi le groupe consistant en : l'antimycine A₀₍ₐ₎, l'antimycine A_{0(b)}, l'antimycine A_{0(c)}, l'antimycine A_{0(d)}, l'antimycine A₁, l'antimycine A₂, l'antimycine A₃, l'antimycine A₄, l'antimycine A₅, l'antimycine A₆, la kitamycine A, la kitamycine B, l'urauchimycine B, la déisovaleryl blastomycine, le dehéxyl-déisovaleryloxylblastomycine, à la condition que le groupe hydroxyle sur le cycle benzène est remplacé par un groupe méthoxy.

2. Utilisation selon la revendication 1, dans laquelle le dérivé antimycine est l'éther de 2-méthoxy antimycine A ou A₃.

3. Utilisation selon la revendication 1 ou 2, dans laquelle le médicament comprend en outre un support pharmaceutique.

4. Utilisation selon la revendication 1, 2 ou 3, dans laquelle le médicament est formulé pour l'administration intraveineuse, sous-cutanée, intramusculaire, intradermale, transdermale, intrathécale, intracérébrale, intrapéritonéale, épidurale ou orale.
